(19) Europäisches Patentamt
European Patent Office
Office européen des brevets

(11) **EP 4 786 949 A1**

(12) **EUROPEAN PATENT APPLICATION**
published in accordance with Art. 153(4) EPC

(43) Date of publication:
05.08.2026 Bulletin 2026/32

(51) International Patent Classification (IPC):
*G01N 1/30* (2006.01)

(21) Application number: 25869606.1

(22) Date of filing: 06.03.2025

(86) International application number:
PCT/CN2025/081014

(87) International publication number:
WO 2026/123477 (18.06.2026 Gazette 2026/25)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 09.12.2024 CN 202411802394

(71) Applicants:
• Jiang, Hongtao
  Shenzhen, Guangdong 518000 (CN)
• Jiang, Hongbo
  Shenzhen, Guangdong 518000 (CN)

(72) Inventors:
• Jiang, Hongtao
  Shenzhen, Guangdong 518000 (CN)
• Jiang, Hongbo
  Shenzhen, Guangdong 518000 (CN)

(74) Representative: Metida
Gyneju str. 16
01109 Vilnius (LT)

(54) **URINE TREATMENT METHOD AND URINE TREATMENT SYSTEM**

(57) The present application relates to urine processing methods and urine processing systems. Specifically, the application discloses a urine processing method, comprising the following steps: S 1. Dehydration processing the urine without prior separation of urinary sediment and supernatant, such that the urine is gradually dehydrated to successively precipitate various types of crystals until all crystals are fully precipitated; S2. Conducting qualitative and/or quantitative analysis on at least one of the target crystals among oxalates, uric acid and urates, phosphates, magnesium ammonium phosphate, and cystine, which have specific crystal morphologies, to obtain target crystal-related parameters. The application also discloses a urine processing system for implementing the aforementioned urine processing method.

FIG. 1

**Description**

CROSS-REFERENCE TO RELATED APPLICATION

This application claims priority to Chinese Patent Application No. 202411802394.2, filed on December 9, 2024, and titled "Urine Processing Method and Urine Processing System", the entire content of which is incorporated herein by reference.

TECHNICAL FIELD

The present application relates to the field of urine testing field, and more particularly to urine processing methods and urine processing systems.

BACKGROUND

The academic community has established that the direct cause and formation process of urinary calculi mainly comprises the following three stages occurring in sequence: 1) concentration of dissolved calculus-forming components in urine becomes supersaturated; 2) precipitation of crystals of the corresponding components; and 3) continued growth and aggregation of crystals to form urinary calculi. Therefore, accurate evaluation and analysis of stages 1) and 2) are of significant importance for prevention and risk assessment of calculus formation.

Currently, the prevention and risk assessment method for urinary calculi that is widely recognized and recommended by the domestic and international medical communities is the 24-hour urine test. This method focuses on detecting the concentration of calculus-forming components, which involves collecting a patient's urine over a continuous 24-hour period and testing, the parameters tested include urine volume, pH value, and the concentration of related metabolites (potassium, calcium, sodium, magnesium, ammonium, chloride, phosphate, sulfate, nitrate, oxalate, citrate, and uric acid, etc.). Testing institutions can calculate degrees of supersaturation of oxalates, phosphates, uric acid, etc., based on specialized procedures. Despite the method's ability to accurately assess the concentration of urolithiasis-related metabolites in the tested 24-hour-collected urine, it has several significant drawbacks as set forth herein below. 1) The test, which involves collecting urine multiple times over 24 hours, only reflects the overall metabolic indicators of the urine during this period and cannot accurately reflect the high calculus-forming risks associated with high concentrations of urine at certain periods, since calculus formation is the result of both the urolithogenesis due to high concentrations of the urolithiasis-related metabolites in urine at different periods of a day and the accumulation of micro-calculus crystallization over a long period.Thus, the overall test of 24-hour-collected urine mainly reflects the general metabolic situation over 24 hours and cannot accurately reflect the urine conditions at different periods of a whole day, for example, the concentration of the urolithiasis-related metabolites may be higher in morning urine. 2) The interpretation of test results is complex, and the assessment of urolithiasis etiology and urolithiasis risk is inaccurate, since the 24-hour urine test only provides the concentration of related substances and cannot directly reflect the risk level of specific calculus components. Even though specialized design methods and calculation processes can determine degrees of supersaturation of oxalates, phosphates and uric acid, etc., the assessment of lithogenic risk based on these calculated supersaturation indices is still affected by urine pH, ionic strength, other small molecules, and organic macromolecules, as urine is a complex mixture of solutions, and the interaction of the lithogenic substances is extremely complex . Accurate assessment is thus difficult, and there is no consistency in the calculation methods and model settings used by different testing institutions abroad. There are no relevant calculation methods domestically. 3) The testing process is complex and costly. The 24-hour urine test involves a wide variety of analytes, including multiple anions, cations, and related molecular substances, which may require different processing methods using various devices for separate detection, such as biochemical methods and chromatography. There is no single processing method that can test everything, and only a very small number of hospitals domestically have all the necessary testing equipment, and yet the calculation of degrees of supersaturation for oxalates, phosphates, and uric acid, etc., has not been carried out. 4) The test is inconvenient and the follow-up is difficult, as the 24-hour urine test requires carrying urine collection containers and collecting urine multiple times over a continuous 24-hour period, which is very inconvenient for daily life and work; additionally, since urine component is affected by diet, activities, climate, and other factors, a single test or a small number of tests cannot accurately assess the risk of calculus formation. This makes it very inconvenient for long-term monitoring of urolithiasis-related urine indicators. Due to these disadvantages, the 24-hour urine test for urolithiasis-related indicators is greatly limited. In developed countries, the usage rate among calculus patients is less than 10%, and it is even less commonly used domestically. This has led to difficulties in conducting and applying urolithiasis etiology screening and urolithiasis risk assessment.

The information included in the background section of the present application, including any references cited herein and their descriptions or discussions, is included solely for technical reference purposes and is not intended to be construed as limiting the scope of the present application.

SUMMARY

In light of the above considerations, there is a need to develop a urine processing method and a urine processing system that can be used for the etiological screening, prevention, and risk assessment of urinary calculi.

In the patent application filed by the inventors of this application on March 6, 2023, with the application number 2023102019482, titled "Urine Processing Method and Urine Processing System," a method for analyzing urine crystallization was proposed. This method involves separating the sediment from the supernatant in urine, analyzing the sediment crystals, and subjecting the supernatant to crystallization processing and analysis, providing beneficial indicators for the etiological analysis, prevention, and risk assessment of urinary calculi.

Based upon the scientific research of the aforementioned patent application, the inventors of this application have continued scientific experiments and technical research and have found that although the urine processing technology and system proposed in the patent application with application number 2023102019482 can indeed effectively analyze the etiology of urinary calculi and assess the risk of calculus formation, with good feasibility and practicality, the step of "separating the sediment from the supernatant in urine" has been proven in extensive practice to not only increase the detection steps and time in terms of operation but also add to the cost and complexity of equipment and structural design in terms of technology. This leads to extended processing and testing times and higher costs. Particularly in medical institutions that require large-scale, multi-batch urine testing, the time and cost associated with the step of "separating the sediment from the supernatant in urine" are relatively high, which is not conducive to large-scale testing.

Therefore, there is an urgent need for an improved urine processing method and an urine processing system that can address the aforementioned defects and deficiencies while achieving the same or better technical effects as the aforementioned invention.

In view of the above and other considerations the present application is proposed.

According to one aspect of this application, a urine processing method is provided that can effectively detect and assess the urolithogenesis-related etiology and urolithogenesis risk at a lower cost, in a shorter time, and with fewer equipment investments. The urine processing method includes the following steps: S1. Dehydration processing the urine without prior separation of urinary sediment and supernatant, that is, without separating liquid and solid phases of the urine, such that the urine is gradually dehydrated to successively precipitate various types of crystals until they are essentially all precipitated; S2. Conducting qualitative and/or quantitative analysis on at least one of target crystals among the crystals selected from oxalates, uric acid and urates, phosphates, magnesium ammonium phosphate, and cystine, which have specific crystal morphologies, to obtain target crystal- related parameters.

According to an embodiment, depending on the specific condition of the urine (such as hematuria, pyuria, bacteriuria, turbid urine, proteinuria, etc.), the urine can be allowed to stand undisturbed for 10-120 minutes, and at least one layer (upper, middle, or lower layer) or multiple layers of urine can be taken for dehydration and crystallization processing. The crystals appearing in each layer can be qualitatively and/or quantitatively analyzed, and comparative analysis of the results from each layer can also be performed.

According to an embodiment, in step S2, when the urine forms target crystals upon crystallization processing, qualitative and/or quantitative analysis is conducted on the target crystals to obtain urine-related parameters, which include the type and/or quantity of the target crystals.

According to an embodiment, prior to the dehydration processing of the urine, it is predetermined whether sediment crystals already exist in the urine. If they do, qualitative and/or quantitative analysis is conducted on the sediment crystals to obtain sediment crystal-related parameters.

According to an embodiment, the sediment crystal-related parameters include the type and/or quantity of the sediment crystals.

According to an embodiment, in step S1, the dehydration processing is carried out progressively to successively precipitate the crystals in the urine. The dehydration processing is continued until all crystals are fully precipitated. During this process, qualitative and/or quantitative analysis is conducted on the newly precipitated crystals other than the sediment crystals, as well as on the existing and unchanged crystals and the existing and growing crystals in the sediment crystals.

According to an embodiment, qualitative and/or quantitative analysis is conducted on at least one of crystals of oxalates, UA (uric acid) and urates, phosphates, magnesium ammonium phosphate and CYS (cystine), using Raman spectroscopy, infrared (IR) spectroscopy, or manual or AI image recognition technology.

According to an embodiment, the qualitative and/or quantitative analysis includes identifying the type of crystals among oxalates, uric acid and urates, phosphates, magnesium ammonium phosphate, and cystine crystals using image recognition technology, comprising: identifying the crystals as oxalates when the image of the crystals is at least one of the shapes including octahedral, equilateral rhombic, elliptical, dumbbell, biconvex columnar, and drum shapes; identifying the crystals as uric acid when the image of the crystals is at least one of the shapes including irregular rhombic, square, blocky, multilayered lamellar, and petal shapes; identifying the crystals as phosphates containing phosphate groups or hydrogen phosphate groups when the image of the crystals is at least one of the shapes including rod, bar, fagot, feather, and chrysanthemum shapes; identifying the crystals as magnesium ammonium phosphate or triple phosphates when the image of the crystals is at least one of the shapes including box-lid, roof, envelope, and coffin-lid shapes; and identifying the crystals as cystine when the image of the crystals is at least one of the shapes including regular hexagonal and multilayered hexagonal shapes.

According to an embodiment, the image recognition technology is manual image recognition or AI image recognition technology.

According to an embodiment, the dehydration processing includes evaporative dehydration.

According to an embodiment, the dehydration processing includes evaporative dehydration accompanied by cooling.

According to an embodiment, the AI image recognition technology is based on a Deep Learning Neural Network Model algorithm.

According to an embodiment, in step S1, if the urine begins to precipitate crystals, the dehydration processing is continued until the vast majority or essentially all of the crystals are precipitated (optionally, recording the appearance and changes of target crystals during the progressive dehydration process until they are stably precipitated).

According to an embodiment, in step S1, if the urine does not precipitate specific target crystals, the dehydration processing is continued until the residual moisture in the urine is 0%.

According to an embodiment, prior to the dehydration processing in step S1, no centrifugation, sedimentation, or filtration processing is performed on the urine.

According to an embodiment, the urine is raw urine.

According to an embodiment, the urine is a single urine sample or a collection of multiple urine samples.

According to an embodiment, the urine can be urine that has undergone dilution treatment.

According to another inventive concept of this application, a urine processing system is provided, comprising: a dehydration processing module, configured to dehydrate urine without prior separation of urinary sediment and supernatant, until various types of crystals are precipitated and fully formed; and a crystal analysis module, configured to conduct qualitative and/or quantitative analysis on at least one of target crystals among the crystals selected from oxalates, uric acid and urates, phosphates, magnesium ammonium phosphate, and cystine, which have specific crystal morphologies, to obtain target crystal- related parameters.

According to an embodiment, the urine processing system is configured to allow the urine to stand undisturbed for 10-120 minutes depending on the specific condition of the urine (such as hematuria, pyuria, bacteriuria, turbid urine, proteinuria, etc.), and at least one layer (upper, middle, or lower layer) or multiple layers of the urine can be taken for dehydration and crystallization processing. The crystals thus appearing in each layer will be qualitatively and/or quantitatively analyzed, and comparative analysis of the results from each layer can also be performed.

According to an embodiment, the urine processing system is configured to predetermine whether sediment crystals already exist in the urine prior to the dehydration processing. If they do, qualitative and/or quantitative analysis is conducted on the sediment crystals to obtain sediment crystal-related parameters.

According to an embodiment, the sediment crystal-related parameters include the type and/or quantity of the sediment crystals.

According to an embodiment, the dehydration processing module is configured to carry out dehydration processing progressively to successively precipitate the crystals in the urine, and the dehydration processing is continued until all crystals are fully precipitated. During this process, qualitative and/or quantitative analysis is conducted on the newly precipitated crystals other than the sediment crystals, as well as on the existing and unchanged crystals and the existing and growing crystals in the sediment crystals.

According to an embodiment, the urine processing system is configured to conduct qualitative and/or quantitative analysis on at least one of oxalates, uric acid and urates, phosphates, magnesium ammonium phosphate, and cystine crystals using Raman spectroscopy, infrared (IR) spectroscopy, or manual or AI image recognition technology.

According to an embodiment, the urine processing system is configured to identify the type of crystals among oxalates, uric acid and urates, phosphates, magnesium ammonium phosphate, and cystine crystals, using image recognition technology, comprising: identifying the crystals as oxalates when the image of the crystals is at least one of the shapes including octahedral, equilateral rhombic, elliptical, dumbbell, biconvex columnar, and drum shapes; identifying the crystals as uric acid when the image of the crystals is at least one of the shapes including irregular rhombic, square, blocky, multilayered lamellar, and petal shapes; identifying the crystals as phosphates containing phosphate groups or hydrogen phosphate groups when the image of the crystals is at least one of the shapes including rod, bar, fagot, feather, and chrysanthemum shapes; identifying the crystals as magnesium ammonium phosphate or triple phosphates when the image of the crystals is at least one of the shapes including box-lid, roof, envelope, and coffin-lid shapes; and identifying the crystals as cystine when the image of the crystals is at least one of the shapes including regular hexagonal and multilayered hexagonal shapes.

According to an embodiment, the image recognition technology is manual image recognition or AI image recognition technology.

According to an embodiment, the multimodal feature extraction module further includes a Deep Learning Neural Network Model.

According to an embodiment, the crystal analysis module includes a crystal image AI recognition subsystem, which includes: a multimodal feature extraction module configured to include a text encoder for outputting text feature vectors and an image encoder for extracting features from input images and outputting image feature vectors; a feature database configured to store the text feature vectors and image feature vectors; an intelligent processing module configured to include an adaptive classification unit and a multi-scale image matching engine supporting image retrieval; and an interactive interface configured to facilitate interaction between the crystal image AI recognition subsystem and a user.

According to an embodiment, the dehydration processing module is configured to perform evaporative dehydration.

According to an embodiment, the dehydration processing module is configured to perform evaporative dehydration accompanied by cooling.

By conducting a simplified processing procedure and technical analysis of urine in practice, this application ultimately can output qualitative and quantitative results of urolithiasis-related crystals in urine, displaying the potential calculus components and formation risks of the urine. This provides a basis for the etiological analysis and prevention of urolithiasis.

According to an embodiment, the urine processing system is configured to allow the urine to stand undisturbed for 10-120 minutes depending on the specific condition of the urine (such as hematuria, bacteriuria, turbid urine, proteinuria, etc.), and at least one layer (upper, middle, or lower layer) or multiple layers of urine can be taken for dehydration and crystallization processing. The crystals appearing in each layer can be qualitatively and/or quantitatively analyzed, and comparative analysis of the results from each layer can also be performed.

According to an embodiment, the crystal analysis module is configured to conduct qualitative and/or quantitative analysis on the target crystals when the urine forms target crystals upon crystallization processing to obtain urine-related parameters, which include the type and/or quantity of the target crystals.

The method and/or the system provided in this application can more directly and accurately analyze the etiology of calculi and assess the risk of calculus formation compared to traditional and existing methods and/or systems. It offers simpler and clearer test results that are more intuitive and easier to interpret. The integrated testing and analysis method results in a more streamlined and efficient testing process. The overall system and equipment costs, as well as the cost per test, are lower, making multiple follow-ups for the disease and long-term monitoring of urolithiasis recurrence more convenient and economical.

BRIEF DESCRIPTION OF THE DRAWINGS

The above-mentioned features and advantages of these embodiments, as well as other features and advantages and the ways in which they are implemented, will become more apparent through reference to the following description in conjunction with the drawings, and the embodiments of the present application can be better understood.

FIG. 1 schematically illustrates a flowchart of a urine processing method according to an embodiment of the present application.

FIG. 2 schematically illustrates an embodiment of a urine processing system for implementing the urine processing method shown in FIG. 1.

FIGs. 3A-3E show common urolithiasis-related crystalline patterns of urinary sediment obtained according to the urine processing method embodiment shown in FIG. 1: oxalates (CaOx), uric acid (UA) and urates, phosphates (CaP), magnesium ammonium phosphate (ST), and cystine (CYS) crystals.

FIGs. 4A-4E show target crystalline patterns of urine after dehydration according to the urine processing method embodiment shown in FIG. 1: oxalates (CaOx), uric acid (UA) and urates, phosphates (CaP), magnesium ammonium phosphate (ST), and cystine (CYS) crystals.

FIGs. 5A-5C show the crystallization status of urine with existing sediment crystals after dehydration according to the urine processing method embodiment shown in FIG. 1. Specifically, FIG. 5A shows an image with original sediment crystals remaining unchanged and newly precipitated crystals; FIGs. 5B and 5C comparatively show images with original sediment crystals continuing to grow and newly precipitated crystals.

DETAILED DESCRIPTION

In the following description of the drawings and specific embodiments, the details of one or more embodiments of the present application will be set forth. From these descriptions, drawings, and claims, the other features, objectives, and advantages of the embodiments of the present application can be clearly understood.

It should be understood that the illustrated and described embodiments are not limited to the details of the construction and arrangement of the components as described below. The illustrated embodiments can be other embodiments that can be implemented or carried out in various ways. For example, features illustrated or described as part of one embodiment can be used in another embodiment to form another embodiment. Therefore, the present application covers such modifications and variations within the scope of the appended claims and their equivalents.

Similarly, it should be understood that the phrases and terms used in the text are for descriptive purposes and should not be considered restrictive. The use of "comprising," "including," or "having" and their variations in this text is intended to be open-ended, including the items listed thereafter and their equivalents, as well as additional items. The term "urinary sediment" refers to the solid phase formed upon separation from the supernatant in urine.

As used herein, the term "crystallization processing" refers to the process of inducing crystal formation. The terms "target crystal (s)" and "sediment crystal (s)" are used to distinguish the source of the crystals; target crystals and sediment crystals may be of the same type or different types of crystals.

A more detailed description of the present application will now be provided below with reference to specific embodiments of the present application.

Embodiment 1

Multiple patients with a history of urolithiasis, patients without urinary calculi, and healthy normal individuals were recruited as controls. Urine samples were collected from these participants, including morning urine, postprandial urine, nocturnal urine, urine from a specific time period, or a mixture of multiple urine samples. The urine samples can be raw urine or a mixture of raw urine, and can also be diluted with distilled water or pure water in a certain proportion.

Without prior separation of urinary sediment and supernatant, the urine was directly dehydrated, and the target crystals in the dehydrated crystals were analyzed to obtain target crystal-related parameters.

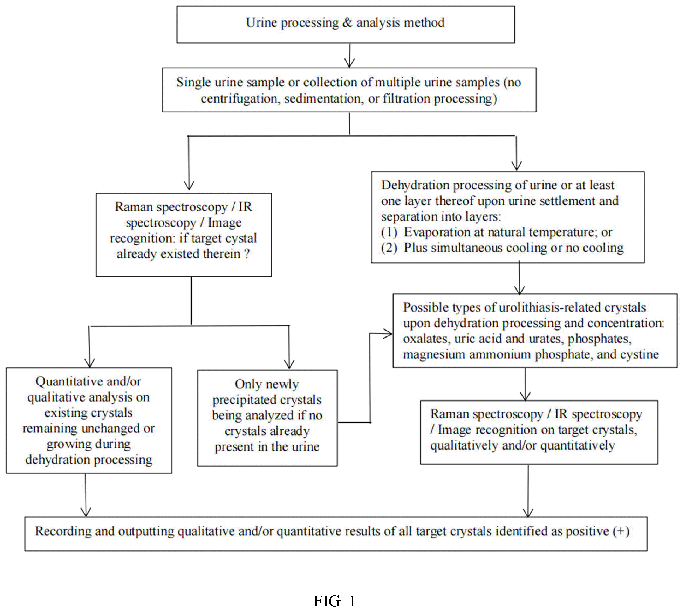

As shown in FIGs. 1 to 4E, an exemplary testing and analysis procedure is described in detail as follows:

1. Selective Dry Chemistry Analysis: The urine may be subjected to dry chemistry analysis to obtain indicators such as pH value, specific gravity, osmotic pressure, and electrical conductivity. Urine dry chemistry indicators play an important role in the formation and recurrence risk control of urinary calculi. For example, an acidic pH value tends to induce the formation of calcium oxalate, uric acid, and cystine calculi, while an alkaline pH value tends to induce the formation of calcium phosphate and magnesium ammonium phosphate calculi. High specific gravity and high osmotic pressure indicate a higher solute concentration in the urine, and high electrical conductivity indicates a higher concentration of electrolytes in the urine. These are risk factors that influence the formation and recurrence of urinary calculi.

2. Dehydration Processing without Prior Separation: The urine is dehydrated without prior separation of urinary sediment and supernatant (e.g., by centrifugation or filtration).

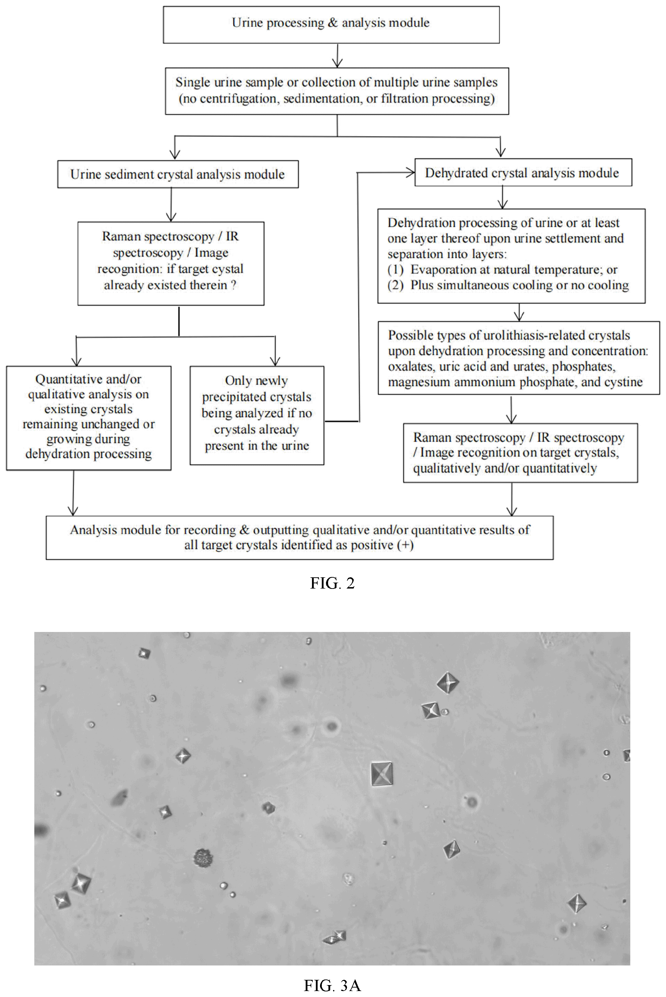

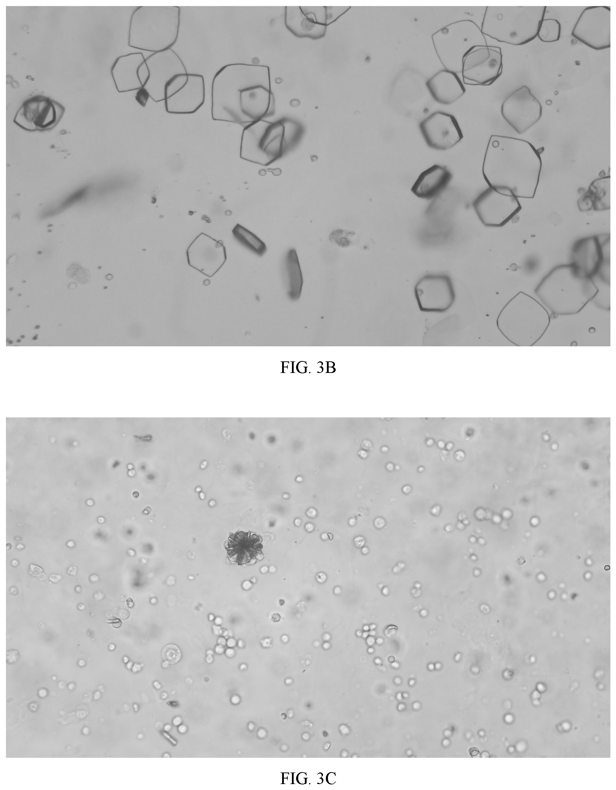

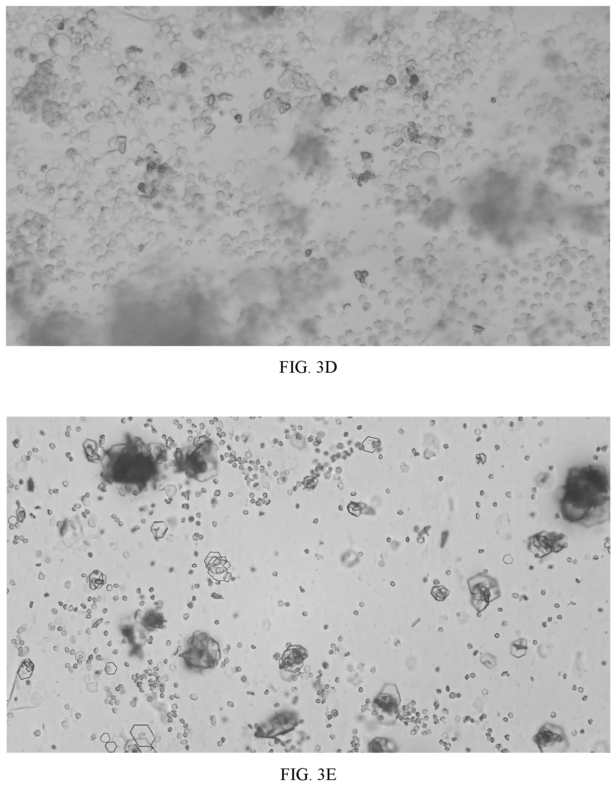

3. Pre-Identification of Sediment Crystals: Prior to dehydration processing, it is predetermined whether sediment crystals already exist in the urine. If they do, qualitative and/or quantitative analysis is conducted on the sediment crystals to obtain parameters related to the target sediment crystals. Sediment crystals in the urinary sediment, if present, will be analyzed qualitatively and quantitatively using image recognition (e.g., through an Olympus Optical Microscope, or a digital camera) or Raman spectroscopy (e.g., through a micro-Raman spectrometer). Qualitative analysis determines the presence of sediment crystals and analyzes the type of sediment crystals (as shown in FIGs. 3A-3E, which display the types of target sediment crystals in urinary sediment. For example, FIG. 3A shows CaOx (calcium oxalate) sediment crystals, which may appear as octahedral, equilateral rhombic, elliptical, or dumbbell-shaped; FIG. 3B shows UA (uric acid) sediment crystals, which may appear as irregular rhombic, square, or blocky; FIG. 3C shows CaP (calcium phosphate) sediment crystals, which may appear as rod-shaped, chrysanthemum-like, bar-shaped, fagot-shaped, or feather-shaped; FIG. 3D shows sediment crystals ST (magnesium ammonium phosphate or triple phosphate), which may appear as roof-shaped, box-lid, envelope, or coffin-lid shapes; FIG. 3E shows CYS (cystine) sediment crystals, which may appear as regular hexagonal or multilayered hexagonal shapes). Quantitative analysis involves counting and measuring the size of target sediment crystals that are positive (+) in qualitative analysis, as well as other statistical treatments, to obtain sediment crystal-related parameters.

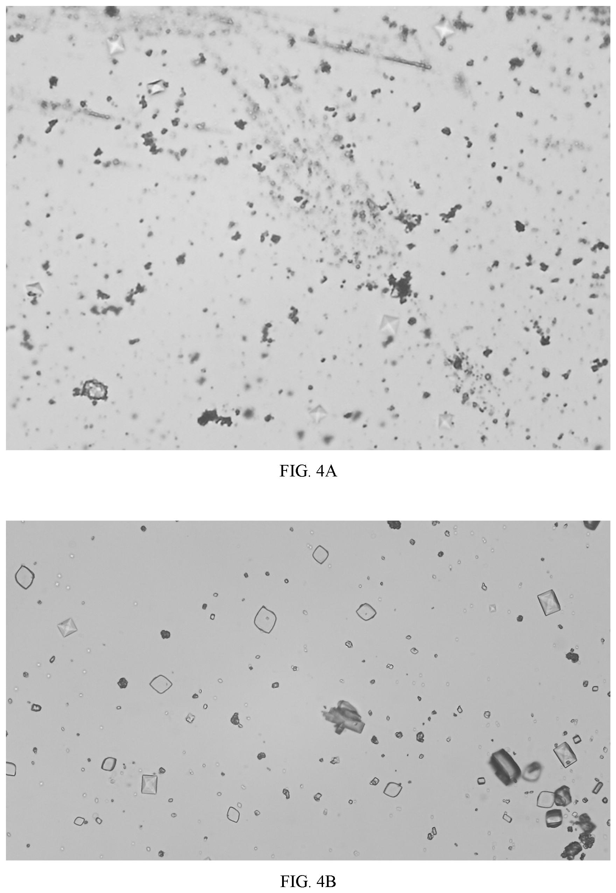

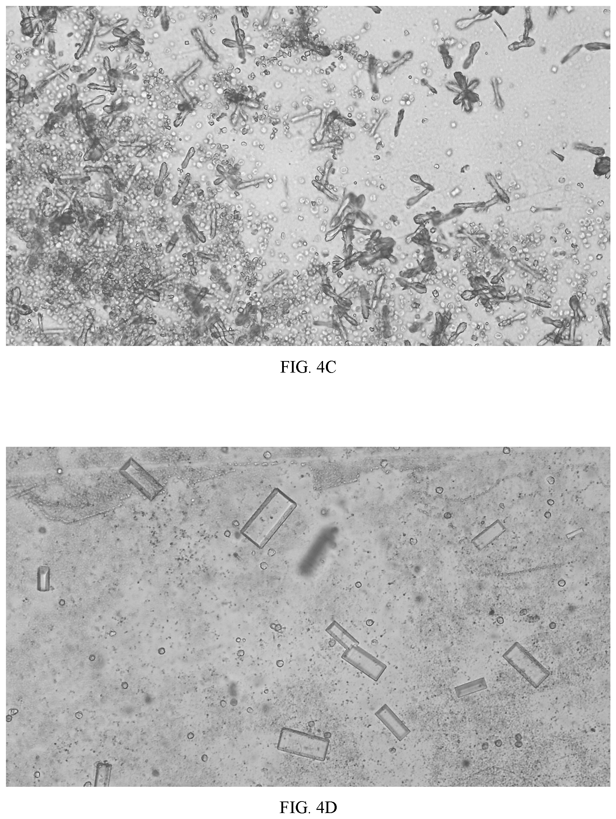

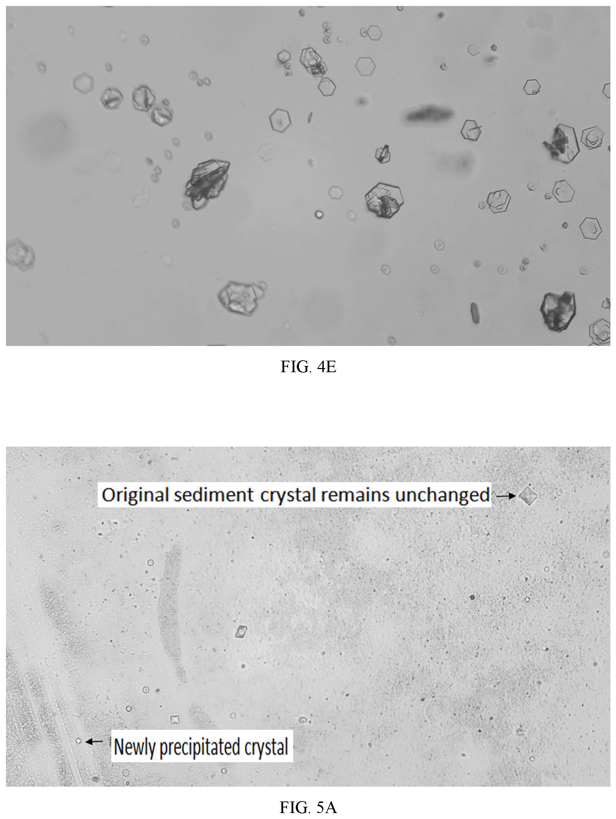

4. Processing and Analysis of Urine: A certain amount of urine is drawn and then evaporated and crystallized at temperatures of 10-37°C, for example, at temperatures of 10°C, 25°C, and 37°C. The urine gradually loses water to form various types of crystals, which may include urolithiasis-related target crystals. The target crystals are subjected to qualitative analysis using Raman spectroscopy, infrared spectroscopy, manual or AI image recognition to identify the type, chemical composition, and crystal structure of the target crystals (as shown in FIGs. 4A-4E, which display the types of urolithiasis-related target crystals in urine. For example, FIG. 4A shows newly precipitated target crystals CaOx, which may appear as octahedral, equilateral rhombic, elliptical, or dumbbell-shaped; FIG. 4B shows newly precipitated target crystals UA (uric acid), which may appear as irregular rhombic, square, or blocky; FIG. 4C shows newly precipitated target crystals CaP, which may appear as rod-shaped, chrysanthemum-like, bar-shaped, fagot-shaped, or feather-shaped; FIG. 4D shows newly precipitated target crystals ST, which may appear as roof-shaped, box-lid, envelope, or coffin-lid shapes; FIG. 4E shows newly precipitated target crystals CYS, which may appear as regular hexagonal or multilayered hexagonal shapes). The urine-related parameters are obtained through image analysis of the target crystals, quantitative counting, size and area proportion of the target crystals, and statistical data analysis.

According to the current research and theoretical consensus in the medical community on the urolithogenesis (such as the references of "International Urolithiasis Alliance: Urolithiasis Metabolism and Management Guidelines 2022"), the urolithogenesis, i.e., the formation of urinary calculi, is closely related to the sediment crystals and the saturation degree of calculus-related components in urine, that is, (1) the sediment crystals in urine sediment are crystals that already exist in urine, indicating that the components of these crystals are already oversaturated in urine (at least locally oversaturated while secreted by local parts of kidney to form urine), and are an important cause of urinary calculus formation, (2) the oversaturation of calculus-related components in supernatant is prone to induce precipitatation and formation of crystals, which is thus closely related to urolithogenesis.

In this embodiment, continuous and timed recording and analysis were performed on the sediment crystals (if sediment crystals already exist) and the situation during the dehydration processing, as well as the newly precipitated crystals that were successively formed and ultimately fully precipitated as the urine was gradually dehydrated. These may include: (1) sediment crystals that already exist and remain unchanged, (2) sediment crystals that already exist and continue to grow, and (3) newly precipitated crystals other than sediment crystals. Qualitative analysis, classification, counting, timing of appearance, final size, and other statistical data and analyses were performed on these three types of target crystals, and the relevant results were output as urolithiasis-related crystalline parameters of the urine.

The qualitative and quantitative data of the existing sediment crystals and the newly precipitated target crystals, as well as the changes and final total amount of crystals, provide a basis for the clinical analysis of the etiology and urolithiasis risk of corresponding calculi component.

The results of this embodiment indicate that: (1) when a certain type of urolithiasis-related crystal already exists in the urinary sediment, it indicates that the urine is in the stage of calculus formation, that is, this type of solid crystal in the urine may further form calculi, and the risk of calculus formation for this component is classified as high risk; (2) if no urolithiasis-related crystals are present in the urinary sediment, rather, urolithiasis-related target crystals appear after urine dehydration, the urine has the potential to form this type of crystal, and the risk of calculus formation for this component is classified as medium risk; (3) when no urolithiasis-related crystals are present in the urine after dehydration processing, while considering that the concentration of urine produced by different parts of each kidney is not completely consistent, there is still possibility of higher concentration urine in some kidney areas, and the possibility of crystal formation cannot be completely ruled out, therefore, the risk of calculus formation is classified as low risk or very low risk. Urine dry chemistry indicators such as pH value, specific gravity, osmotic pressure, and electrical conductivity are auxiliary factors and factors that promote or inhibit the formation of a specific type of crystal and its subsequent induction of calculus formation, thus are for reference and auxiliary judgment only.

Embodiment 2

The implementation mode and steps of Embodiment 2 are essentially the same as those of Embodiment 1, with the difference being that, in the step corresponding to step 2 of Embodiment 1, no prior separation of urinary sediment and supernatant is performed, that is, no separation of the liquid and solid phases of urine is carried out. However, the urine is allowed to stand undisturbed for a short period to stratify (e.g., for approximately 10-120 minutes, etc.), and then the upper, middle, or lower layer of urine is taken, or alternatively urine samples from different layers are taken for separate treatment, according to the specific condition of the urine (such as hematuria, pyuria, bacteriuria, proteinuria, turbid urine, etc.). Single-layer analysis can be performed, or multi-layer comparative analysis can be chosen to provide more comprehensive and accurate crystallization data, which is also beneficial for crystal image recognition.

Additionally, in this Embodiment 2, continuous and timed recording and analysis are performed on the sediment crystals (if existed) and their status during the dehydration processing, as well as the newly precipitated crystals that are successively formed and ultimately fully precipitated as the urine is gradually dehydrated. These may include: (1) sediment crystals that already exist and remain unchanged, (2) sediment crystals that already exist and continue to grow, and (3) newly precipitated crystals other than sediment crystals. Qualitative, classification, counting, timing of appearance, final size, and other statistical data and analyses are performed on these three types of target crystals, and the relevant results are output as urolithiasis-related crystalline parameters of the urine. Furthermore, (4) urine samples taken from different layers are treated separately, and individual analysis or comparative analysis can be chosen to provide more comprehensive and accurate crystallization data, which is also beneficial for crystal image recognition.

The qualitative and quantitative data of the existing sediment crystals and the newly precipitated target crystals, as well as the changes and final size, shape, and total amount of crystals, provide a basis for the clinical analysis of the etiology and urolithiasis risk of corresponding calculi component.

The remaining steps are essentially the same as those in Embodiment 1 and will not be reiterated one by one.

Embodiment 3

Embodiment 3 is essentially the same as Embodiment 1, with the difference being that, in step 3, when sediment crystals have already been identified in the urine, dehydration processing of the urine will be continued. In this case, the following two scenarios may occur:

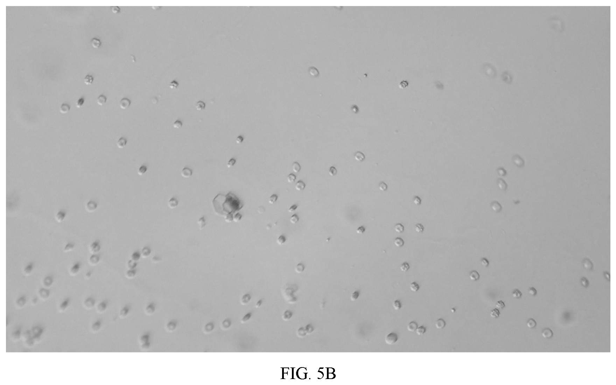

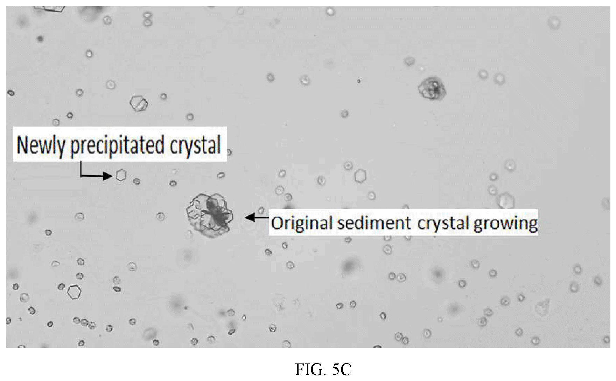

(1) The original sediment crystals remain unchanged, but new crystals are precipitated, as shown in FIG. 5A.
(2) The original sediment crystals continue to grow, and new crystals are precipitated simultaneously, as shown in FIGs. 5B and 5C.

In these two scenarios, qualitative and/or quantitative analysis is performed on the unchanged/continuously growing sediment crystals and the newly precipitated crystals to obtain parameters related to these crystals. Qualitative and quantitative analysis will be performed using image recognition (e.g., through an Olympus Optical Microscope and digital camera) or Raman spectroscopy (e.g., through a micro-Raman spectrometer). Qualitative analysis determines the presence and type of the aforementioned crystals and analyzes the type of crystals (as specifically shown in FIGs. 5A-5C) to obtain urine-related parameters.

Embodiment 4

According to one embodiment of the present application, a urine processing system is also provided. The modules of the system and the performed steps can be seen in FIG. 2. The urine processing system may include:

(1) A dehydration processing module, configured to dehydrate urine without prior separation of urinary sediment and supernatant, until various types of crystals are precipitated and fully formed.

(2) A crystallization analysis module, configured to conduct qualitative and/or quantitative analysis on at least one of the target crystals among oxalates, uric acid and urates, phosphates, magnesium ammonium phosphate, and cystine, which have specific crystal morphologies, to obtain target crystal- related parameters.

The dehydration processing module may be configured to allow the urine to stand undisturbed for a short period, such as several hours, and more preferably for 10-120 minutes, to stratify the urine. At least the upper layer and/or the lower layer of urine will be processed, or alternatively multiple layers of urine will be processed, to gradually dehydrate the urine and successively precipitate various types of crystals until they are fully formed.

The urine processing system may be configured to predetermine whether sediment crystals already exist in the urine prior to dehydration processing. If they do, qualitative and/or quantitative analysis is conducted on the sediment crystals to obtain sediment crystal-related parameters.

The sediment crystal-related parameters of the target sediment crystals may include the type and/or quantity of the sediment crystals.

The dehydration processing module may be configured to carry out dehydration processing progressively to successively precipitate the crystals in the urine, and the dehydration processing is continued until all crystals are fully precipitated. The dehydration processing module may further be configured to conduct qualitative and/or quantitative analysis on the newly precipitated crystals upon dehydration processing other than the sediment crystals, as well as on the existing and unchanged crystals and the existing and growing crystals in the sediment crystals.

The urine processing system may be configured to conduct qualitative and/or quantitative analysis on at least one of oxalates, uric acid and urates, phosphates, magnesium ammonium phosphate, and cystine crystals using Raman spectroscopy, infrared spectroscopy, or manual or AI image recognition technology.

The urine processing system may be configured to identify the type of crystals among oxalates, uric acid and urates, phosphates, magnesium ammonium phosphate, and cystine crystals using image recognition technology, comprising: identifying the crystals as oxalates when the image of the crystals is at least one of the shapes including octahedral, equilateral rhombic, elliptical, dumbbell, biconvex columnar, and drum shapes; identifying the crystals as uric acid when the image of the crystals is at least one of the shapes including irregular rhombic, square, blocky, multilayered lamellar, and petal shapes; identifying the crystals as phosphates containing phosphate groups or hydrogen phosphate groups when the image of the crystals is at least one of the shapes including rod, bar, fagot, feather, and chrysanthemum shapes; identifying the crystals as magnesium ammonium phosphate or triple phosphates when the image of the crystals is at least one of the shapes including box-lid, roof, envelope, and coffin-lid shapes; and identifying the crystals as cystine when the image of the crystals is at least one of the shapes including regular hexagonal and multilayered hexagonal shapes.

The dehydration processing module may be configured to perform evaporative dehydration.

The dehydration processing module may be configured to perform evaporative dehydration accompanied by cooling.

The identification technology for crystal images may be manual image recognition or AI image recognition technology.

Regarding AI image recognition technology, the crystallization analysis module may further include a Deep Learning Neural Network Model for AI image recognition, which implements the AI image recognition technology based on algorithms. For example, a database module for the Deep Learning Neural Network Model may be established, which is configured to store / contain existing data, especially data that has been machine-trained, including a large amount of urine crystal information from the applicants' existing sources, as well as from related medical institutions, physical examination organizations, medical literature, and databases, particularly those including crystal patterns and identified crystal names (types). An image acquisition module for the Deep Learning Neural Network Model may be established, which is configured to acquire crystal patterns/images (including captured crystal images and data, etc.) through devices such as optical microscopes, digital cameras, and cameras of micro-Raman spectrometers, and store them. An image cut and feature extraction module for the Deep Learning Neural Network Model may be established, which is configured to cut individual crystal images from the crystal patterns/images, extract features, and perform preprocessing such as size transformation, normalization, and standardization to extract crystal image features. A feature matching module for the Deep Learning Neural Network Model may be established, which may compare and match the extracted crystal image/pattern features with the stored urine crystal information features in the database to achieve matching and identification of crystals on image/pattern and name/type thereof, enabling rapid and precise AI image recognition of crystals, significantly improving recognition efficiency and accuracy, and reducing manual labor.

An Example of AI Recognition of Urine Crystal Images

According to an example of the AI recognition technology for urine crystal images/patterns of the present invention, a crystal image AI recognition system based on multimodal deep learning is adopted. This system uses a large number of classified crystal patterns from existing urine processing as training data for model training and learning (e.g., deep learning). After the model training is completed, the crystal image AI recognition system (e.g., its Deep Learning Neural Network Model) can assist in identifying the classification of the crystal pattern/image based on user input images, and can selectively provide several of the most similar crystal patterns/images to aid in further manual verification and judgment, as well as other functions. This example can be optionally applied to the aforementioned embodiments.

I. Important Modules/Components of the Crystal Image/Pattern AI recognition System in This Example

1) Multimodal Feature Extraction Module: Equipped with an NVIDIA A100 GPU cluster (8 nodes), supporting mixed-precision training and FP16 inference acceleration. The multimodal feature extraction module adopts an improved CLIP (Contrastive Language-Image Pretraining) model architecture, comprising:

- Text Encoder: A TextModel based on the Transformer architecture, featuring 12 layers of self-attention mechanisms, outputting a text feature vector with dimension of 512.
- Image Encoder: An ImageModel based on the ViT-B/16 structure, segmenting input images into 16×16 pixel patches for feature extraction, outputting an image feature vector with dimension of 512.

2) Feature Database: Utilizing a distributed Milvus 2.1 vector database, supporting near real-time search for billion-level high-dimensional vectors. The implementation details of the feature database include:

- Milvus Cluster Configuration:

  - Deployed in a 3-node distributed setup (16 cores/64GB memory/2TB SSD);
  - Index type: HNSW (Hierarchical Navigable Small World) index.

3) Intelligent Processing Module, comprising:

- Adaptive Classification Unit: An integrated hybrid classifier combining cosine similarity and K-nearest neighbors algorithm (K=5);
- Multiscale Image Matching Engine: Supporting multi-metric space search with L2 distance and IP inner product.

4) Interactive Interface: Providing dual-channel access via RESTful API and Web-based visualization interface.

II. Data Processing Workflow of the crystal image AI recognition System in This Example

Step 1: Multimodal Model Optimization

- Domain Adaptation of Pretrained CLIP Model:

  - Fine-tuning on authoritative and specialized medical imaging and image datasets (including urine crystal images and corresponding crystal categories as well as pathological/etiologic description texts, for example, from domestic and international medical literature, image databases, experimental data and images from the inventors and their team of this application, etc.).
  - Employing a contrastive learning strategy with the loss function:

$$L = 0.5 \times (L\_image\text{-}text + L\_text\text{-}image)$$

Wherein, the cross-modal loss function uses temperature-scaled cross-entropy with a temperature parameter, $\tau =$ 0.07.

Step 2: Knowledge Base Construction

1) Category Label Processing:

- Pre-defining 7 major categories with 42 subcategories of crystal labels (e.g., "Hexagonal uric acid crystals [code UA-6]", "Needle-shaped calcium oxalate crystals [code OX-1]").
- Generating standardized descriptive texts for each label (e.g., "Rhombic crystals exhibiting typical birefringence characteristics with a length-to-width ratio > 3:1").
- Extracting label text feature vectors $FT \in R^{512}$ using the TextModel.

2) Image Data Ingestion:

- Data Sources: Including but not limited to the urine research center of Guangzhou Youjing Medical Co., Ltd., other medical testing centers, National Medical Imaging Database (NMID), domestic and international medical literature, internet-related materials, and direct connection with LIS systems and microscope digital imaging devices.
- Preprocessing Flow: Raw image $\rightarrow$ Non-uniform illumination correction $\rightarrow$ Adaptive threshold segmentation $\rightarrow$ ROI extraction $\rightarrow$ Resolution normalization (512$\times$512).
- Feature Extraction: Obtaining image feature vectors $FI \in R^{512}$ using the ImageModel.
- Feature Enhancement: Implementing PCA dimensionality reduction (512$\rightarrow$256) plus L2 normalization on feature vectors.

Step 3: Intelligent Retrieval and Classification

1) Image-to-Image Search Workflow:

- Extracting feature vector Q_img from the input image after preprocessing.
- Executing Approximate Nearest Neighbor (ANN) search in the Milvus database:
  Search conditions: top_k=10, metric_type="IP".
- Result optimization: Applying Density-Based Spatial Clustering of Applications with Noise (DBSCAN) algorithm to filter out anomalous matches.

2) Multimodal Retrieval (e.g., searching images by category, searching images by text, etc.):

- Supporting hybrid queries: "Find uric acid crystals matching the description of 'hexagonal crystals'."
- Implementation way:

$$Q\_hybrid = \alpha \times FT\_text + \beta \times FI\_image\ (\alpha + \beta = 1)$$

III. Testing and Validation

Testings are deployed at Guangzhou Youjing Medical Co., Ltd., as follows:

- Test Dataset: Comprising 15,827 clinical sample images.
- Performance Metrics:

  - Classification accuracy: Top-1 reaches 92.7%; Top-5 reaches 98.3%.
  - Retrieval time: Average 83ms per query (with GPU acceleration).
  - Reduction in manual review workload by 95%.

IV. Typical Application Scenarios of the crystal image AI recognition System in This Example

Some typical application scenarios include:

1. Urologists and laboratory technicians upload urine crystal images from microscope fields of view, and the system automatically labels suspicious crystal types.
2. In teaching systems, students upload recognition results, and the system returns the top 5 most similar typical case images.
3. Researchers retrieve specific morphological feature crystal cases through natural language descriptions.
4. Users from physical examination organization or other testing institutions upload urine crystal images, and the system automatically labels suspicious crystal types and provides assessments and possible warnings.

V. Specific Technical Effects of the crystal image AI recognition System in This Example

The crystal image AI recognition system in this example achieves technological advantages through at least the following innovations:
1) Establishing a joint embedding space of urine crystal visual features and clinical descriptive texts through a multimodal alignment strategy.
2) Supporting the addition of new crystal types by fine-tuning only the classification layer (freezing the backbone network) through a dynamic incremental learning mechanism.
3) Combining the advantages of Exact Search and ANN to balance retrieval accuracy and efficiency, significantly saving manual time and labor while greatly improving the recognition accuracy and efficiency of crystal images through a hybrid retrieval architecture.
4) Compared with traditional manual recognition methods, the crystal image AI recognition system in this example provides the following superior technical effects as shown in the table below:

| Crystal Detection Indicator | Traditional Manual Recognition | This System's AI Recognition | Improvement of This System's AI Recognition over Manual Recognition |
|---|---|---|---|
| Single-crystal sample processing time | 3-5 minutes | < 2 seconds | 99%↑ |
| Accuracy of crystal image/morphology classification | 78.2% | 93.5% | 19.6%↑ |
| Accuracy of crystal quantification | 60-70% | 98% | 40-63.3%↑ |
| Recognition rate of abnormal morphology/rare urine crystals | 62.4% | 89.1% | 42.8%↑ |

In summary, according to the present invention, a specific processing procedure and technical analysis of urine that are simplified in practice (e.g., including manual recognition and/or AI recognition of crystal images, as well as analysis of other parameters) can ultimately output qualitative and quantitative results of urolithiasis-related crystals in urine, thereby indicating the urolithiasis risk of the urine and providing basis for the etiological analysis and prevention of urolithiasis.

According to one example, the urine processing system may be configured to allow the urine to stand undisturbed for a period of time, depending on the specific condition of the urine (such as hematuria, bacteriuria, turbid urine, proteinuria, etc.), for example, within 3 hours, or for 10-120 minutes, and to take at least one layer (upper, middle, or lower layer) or multiple layers of urine, for dehydration and crystallization processing. Qualitative and/or quantitative analysis of the crystals appearing in each layer can be performed, and comparative analysis of the results from each layer can also be conducted.

According to one example, the crystallization analysis module may be configured to conduct qualitative and/or quantitative analysis on the target crystals when the urine forms target crystals upon crystallization processing to obtain urine-related parameters, which include the type and/or quantity of the target crystals.

The improved urine processing method and urine processing system of the present application have been proven in extensive practice to not only greatly and substantially reduce the testing steps and time in terms of operation, but also reduce the cost and complexity of the required equipment and related structural design in terms of technology. This shortens the overall processing and testing time, reduces the cost and expenses of equipment and processing steps, and thus overcomes the defects and deficiencies of the existing technology and achieves better technical effects than the existing technology.

For more specific examples and embodiments of the processes, systems, devices or apparatuses, and processing steps of the urine processing method and urine processing system of the present application, reference may be made to the urine processing and analysis methods and systems, including the equipment, components, processing methods, and related steps, disclosed in the Chinese patent application with application number 2023102019482, filed by the same inventors on March 6, 2023, titled "Urine Processing Method and Urine Processing System." The content of that Chinese patent application related to the present application is incorporated herein by reference. In particular, in addition to the crystal images/patterns shown in FIGs. 3A-3E, 4A-4E, and 5A-5C, those crystal images/patterns that may appear during the crystallization process and are shown in the application 2023102019482 are also incorporated by reference into the present application, including their images/patterns, names, and judgment ways, for use in manual image recognition, AI image recognition, and data training, etc., as if specifically described and disclosed in the present application.

The foregoing description of several embodiments of the present application is provided for illustrative purposes. The description is not intended to be exhaustive, nor is it intended to limit the application to the precise parameters, values, steps, and/or forms disclosed herein. It will be apparent to those skilled in the art that many modifications and variations are possible in light of the above teachings. The technical features, parameters, and numerical values of the above embodiments can be combined in other feasible ways, provided that they do not contradict each other and can be implemented. For the sake of brevity, not all possible combinations of the technical features of the aforementioned embodiments have been described. However, as long as there are no contradictions in the combinations of these technical features, parameters, and numerical values, they should be considered within the scope of the present disclosure. The scope of the application and all equivalents are intended to be defined by the appended claims.

**Claims**

1. A urine processing method, comprising the steps of:

12

S1. Dehydration processing the urine without prior separation of urinary sediment and supernatant, such that the urine is gradually dehydrated to successively precipitate various types of crystals until all crystals are fully precipitated; and

S2. Conducting qualitative and/or quantitative analysis on at least one of the target crystals among oxalates, uric acid and urates, phosphates, magnesium ammonium phosphate, and cystine, which have specific crystal morphologies, to obtain target crystal- related parameters.

2. The urine processing method according to claim 1, wherein in step S1, the urine is allowed to stand undisturbed for 10-120 minutes to stratify, and at least the upper layer and/or the lower layer of urine is processed, or multiple layers of urine are processed, such that the urine is gradually dehydrated to successively precipitate various types of crystals until all crystals are fully precipitated.

3. The urine processing method according to any one of claims 1-2, wherein in step S2, when the urine forms target crystals upon crystallization processing, qualitative and/or quantitative analysis is conducted on the target crystals to obtain urine-related parameters, the urine-related parameters including the type and/or quantity of the target crystals.

4. The urine processing method according to any one of claims 1-3, wherein prior to the dehydration processing of the urine, predetermining whether sediment crystals already exist in the urine, and if they do, qualitative and/or quantitative analysis is conducted on the sediment crystals to obtain sediment crystal-related parameters.

5. The urine processing method according to claim 4, wherein the sediment crystal-related parameters include the type and/or quantity of the sediment crystals.

6. The urine processing method according to claim 4 or 5, wherein in step S1, the dehydration processing is carried out progressively to successively precipitate the crystals in the urine; and the dehydration processing is continued until all crystals are fully precipitated; wherein record is continuously made and qualitative and/or quantitative analysis is conducted on the newly precipitated crystals other than the sediment crystals during the dehydration processing, as well as on the existing and unchanged crystals and the existing and growing crystals in the sediment crystals.

7. The urine processing method according to any one of claims 1-6, wherein qualitative and/or quantitative analysis is conducted on at least one of oxalates, uric acid and urates, phosphates, magnesium ammonium phosphate, and cystine crystals using Raman spectroscopy, infrared spectroscopy, or manual or AI image recognition technology.

8. The urine processing method according to claim 7, wherein the qualitative and/or quantitative analysis includes identifying the type of crystals among oxalates, uric acid and urates, phosphates, magnesium ammonium phosphate, and cystine crystals using image recognition technology, comprising:

identifying the crystals as oxalates when the image of the crystals is at least one of the shapes including octahedral, equilateral rhombic, elliptical, dumbbell, biconvex columnar, and drum shapes;
identifying the crystals as uric acid when the image of the crystals is at least one of the shapes including irregular rhombic, square, blocky, multilayered lamellar, and petal shapes;
identifying the crystals as phosphates containing phosphate groups or hydrogen phosphate groups when the image of the crystals is at least one of the shapes including rod, bar, fagot, feather, and chrysanthemum shapes;
identifying the crystals as magnesium ammonium phosphate or triple phosphates when the image of the crystals is at least one of the shapes including box-lid, roof, envelope, and coffin-lid shapes; and
identifying the crystals as cystine when the image of the crystals is at least one of the shapes including regular hexagonal and multilayered hexagonal shapes.

9. The urine processing method according to any one of claims 1-8, wherein the image recognition technology is manual image recognition or AI image recognition technology.

10. The urine processing method according to any one of claims 1-9, wherein the dehydration processing includes evaporative dehydration.

11. The urine processing method according to any one of claims 1-10, wherein the dehydration processing includes evaporative dehydration accompanied by cooling.

12. The urine processing method according to claim 9, wherein the AI image recognition technology is based on a Deep Learning Neural Network Model algorithm.

13. A urine processing system, comprising:

a dehydration processing module configured to dehydrate urine without prior separation of urinary sediment and supernatant until various types of crystals are fully precipitated; and

a crystal analysis module configured to conduct qualitative and/or quantitative analysis on at least one of the target crystals among oxalates, uric acid and urates, phosphates, magnesium ammonium phosphate, and cystine, which have specific crystal morphologies, to obtain target crystal- related parameters.

14. The urine processing system according to claim 13, wherein the dehydration processing module is configured to allow the urine to stand undisturbed for 10-120 minutes to stratify, and at least the upper layer and/or the lower layer of urine is processed, or multiple layers of urine are processed, such that the urine is gradually dehydrated to successively precipitate various types of crystals until all crystals are fully precipitated.

15. The urine processing system according to any one of claims 13-14, wherein the crystal analysis module is configured to conduct qualitative and/or quantitative analysis on the target crystals when the urine forms target crystals upon crystallization processing to obtain urine-related parameters, the urine-related parameters including the type and/or quantity of the target crystals.

16. The urine processing system according to any one of claims 13-15, wherein the urine processing system is configured to predetermine whether sediment crystals already exist in the urine prior to the dehydration processing, and if they do, qualitative and/or quantitative analysis is conducted on the sediment crystals to obtain sediment crystal-related parameters.

17. The urine processing system according to claim 16, wherein the sediment crystal-related parameters include the type and/or quantity of the sediment crystals.

18. The urine processing system according to claim 16 or 17, wherein the dehydration processing module is configured to carry out dehydration processing progressively to successively precipitate the crystals in the urine, and the dehydration processing is continued until all crystals are fully precipitated; wherein the dehydration processing module is further configured to continuously record and conduct qualitative and/or quantitative analysis on the newly precipitated crystals other than the sediment crystals during the dehydration processing, as well as on the existing and unchanged crystals and the existing and growing crystals in the sediment crystals.

19. The urine processing system according to any one of claims 13-18, wherein the urine processing system is configured to conduct qualitative and/or quantitative analysis on at least one of oxalates, uric acid and urates, phosphates, magnesium ammonium phosphate, and cystine crystals using Raman spectroscopy, infrared spectroscopy, or manual or AI image recognition technology.

20. The urine processing system according to claim 19, wherein the urine processing system is configured to identify the type of crystals among oxalates, uric acid and urates, phosphates, magnesium ammonium phosphate, and cystine crystals using image recognition technology, comprising:

identifying the crystals as oxalates when the image of the crystals is at least one of the shapes including octahedral, equilateral rhombic, elliptical, dumbbell, biconvex columnar, and drum shapes;

identifying the crystals as uric acid when the image of the crystals is at least one of the shapes including irregular rhombic, square, blocky, multilayered lamellar, and petal shapes;

identifying the crystals as phosphates containing phosphate groups or hydrogen phosphate groups when the image of the crystals is at least one of the shapes including rod, bar, fagot, feather, and chrysanthemum shapes;

identifying the crystals as magnesium ammonium phosphate or triple phosphates when the image of the crystals is at least one of the shapes including box-lid, roof, envelope, and coffin-lid shapes; and

identifying the crystals as cystine when the image of the crystals is at least one of the shapes including regular hexagonal and multilayered hexagonal shapes.

21. The urine processing system according to any one of claims 13-20, wherein the image recognition technology is manual image recognition or AI image recognition technology.

22. The urine processing system according to claim 21, wherein the crystal analysis module includes a crystal image AI recognition subsystem, the crystal image AI recognition subsystem comprising:

    a multimodal feature extraction module configured to include a text encoder for outputting text feature vectors and an image encoder for extracting features from input images and outputting image feature vectors;
    a feature database configured to store the text feature vectors and the image feature vectors;
    an intelligent processing module configured to include an adaptive classification unit and a multi-scale image matching engine supporting image retrieval; and
    an interactive interface configured to facilitate interaction between the crystal image AI recognition subsystem and a user.

23. The urine processing system according to any one of claims 13-22, wherein the dehydration processing module is configured to perform evaporative dehydration.

24. The urine processing system according to any one of claims 13-23, wherein the dehydration processing module is configured to perform evaporative dehydration accompanied by cooling.

25. The urine processing method according to claim 22, wherein the multimodal feature extraction module further includes a Deep Learning Neural Network Model.

FIG. 1

Urine processing & analysis module

↓

Single urine sample or collection of multiple urine samples
(no centrifugation, sedimentation, or filtration processing)

Urine sediment crystal analysis module

Dehydrated crystal analysis module

Raman spectroscopy / IR spectroscopy / Image recognition: if target cystal already existed therein ?

Dehydration processing of urine or at least one layer thereof upon urine settlement and separation into layers:
(1) Evaporation at natural temperature; or
(2) Plus simultaneous cooling or no cooling

Quantitative and/or qualitative analysis on existing crystals remaining unchanged or growing during dehydration processing

Only newly precipitated crystals being analyzed if no crystals already present in the urine

Possible types of urolithiasis-related crystals upon dehydration processing and concentration: oxalates, uric acid and urates, phosphates, magnesium ammonium phosphate, and cystine

Raman spectroscopy / IR spectroscopy / Image recognition on target crystals, qualitatively and/or quantitatively

Analysis module for recording & outputting qualitative and/or quantitative results of all target crystals identified as positive (+)

FIG. 2

FIG. 3A

FIG. 3B

FIG. 3C

FIG. 3D

FIG. 3E

FIG. 4A

FIG. 4B

FIG. 4C

FIG. 4D

FIG. 4E

FIG. 5A

FIG. 5B

FIG. 5C

## INTERNATIONAL SEARCH REPORT

| International application No. |
| --- |
| **PCT/CN2025/081014** |

| A. | CLASSIFICATION OF SUBJECT MATTER |
| --- | --- |
| | G01N1/30(2006.01)i |

According to International Patent Classification (IPC) or to both national classification and IPC

| B. | FIELDS SEARCHED |
| --- | --- |

Minimum documentation searched (classification system followed by classification symbols)

IPC: G01N

Documentation searched other than minimum documentation to the extent that such documents are included in the fields searched

Electronic data base consulted during the international search (name of data base and, where practicable, search terms used)

CNABS, CNTXT, VEN: 尿, 处理, 测, 分析, 脱水, 尿沉渣, 结晶, 上清液, 图像识别, urine, treatment, detect+, urinary sediment, supernate

| C. | DOCUMENTS CONSIDERED TO BE RELEVANT | |
| --- | --- | --- |

| Category* | Citation of document, with indication, where appropriate, of the relevant passages | Relevant to claim No. |
| --- | --- | --- |
| Y | CN 118603683 A (JIANG HONGTAO et al.) 06 September 2024 (2024-09-06) description, paragraphs 5-38 | 1-25 |
| Y | CN 1645139 A (CHANGCHUN DIRUI INDUSTRIAL CO., LTD.) 27 July 2005 (2005-07-27) description, pages 1-4 | 1-25 |
| A | CN 110473167 A (HARBIN ENGINEERING UNIVERSITY) 19 November 2019 (2019-11-19) entire document | 1-25 |
| A | CN 109190590 A (SHENZHEN MEIQIAO MEDICAL TECHNOLOGY CO., LTD.) 11 January 2019 (2019-01-11) entire document | 1-25 |
| A | CN 118172305 A (JIANGSU UNIVERSITY) 11 June 2024 (2024-06-11) entire document | 1-25 |
| A | JP H06138120 A (HITACHI, LTD.) 20 May 1994 (1994-05-20) entire document | 1-25 |

☑ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **28 August 2025** | **29 August 2025** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**EP 4 786 949 A1**

### INTERNATIONAL SEARCH REPORT

International application No.

**PCT/CN2025/081014**

**C. DOCUMENTS CONSIDERED TO BE RELEVANT**

| Category* | Citation of document, with indication, where appropriate, of the relevant passages | Relevant to claim No. |
|---|---|---|
| A | JP 2012132850 A (SYSMEX CORP.) 12 July 2012 (2012-07-12)<br>entire document | 1-25 |

Form PCT/ISA/210 (second sheet) (July 2022)

**INTERNATIONAL SEARCH REPORT**
Information on patent family members

International application No.

**PCT/CN2025/081014**

| Patent document cited in search report | | | Publication date (day/month/year) | Patent family member(s) | | | Publication date (day/month/year) |
|---|---|---|---|---|---|---|---|
| CN | 118603683 | A | 06 September 2024 | None | | | |
| CN | 1645139 | A | 27 July 2005 | None | | | |
| CN | 110473167 | A | 19 November 2019 | None | | | |
| CN | 109190590 | A | 11 January 2019 | None | | | |
| CN | 118172305 | A | 11 June 2024 | None | | | |
| JP | H06138120 | A | 20 May 1994 | None | | | |
| JP | 2012132850 | A | 12 July 2012 | JP | 5727217 | B2 | 03 June 2015 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202411802394
- WO 2023102019482 A
- CN 2023102019482